# EUROPÄISCHE PATENTSCHRIFT

(11) **EP 4 337 049 B1**
(45) Veröffentlichungstag und Bekanntmachung des Hinweises auf die Patenterteilung: **28.08.2024**
(21) Anmeldenummer: 23717403.2
(22) Anmeldetag: 29.03.2023
(51) Int. Cl.: A41D 1/00, B29C 64/118, B29C 64/209, B33Y 30/00, B33Y 10/00

(54) **VERFAHREN ZUM AUFBRINGEN VON ELASTOMER UND EINEM KABEL AUF EINE STOFFLAGE**
METHOD FOR APPLYING ELASTOMER AND A CABLE TO A MATERIAL LAYER
PROCÉDÉ D'APPLICATION D'ÉLASTOMÈRE ET D'UN CÂBLE SUR UNE COUCHE DE MATÉRIAU

(30) Priorität: 14.04.2022 DE 102022109327
(43) Veröffentlichungstag der Anmeldung: 20.03.2024
(73) Patentinhaber: NTT New Textile Technologies GmbH, 72336 Balingen (DE)
(72) Erfinder: BAUER, Hans, 73226 Balingen (DE)
(74) Vertreter: Müller, Gottfried
(86) Internationale Anmeldenummer: PCT/EP2023/025140
(87) Internationale Veröffentlichungsnummer: WO 2023/198309

(56) Entgegenhaltungen:
- WO-A1-2017/091154
- US-A1- 2019 009 472

## Beschreibung

Die Erfindung bezieht sich auf ein Verfahren zum Aufbringen von Elastomer und einem in das Elastomer eingebetteten Kabel auf eine Stofflage eines Bekleidungsstücks oder einer Bandage.

Aus der EP 3 172 977 A1 ist ein Verfahren zur Herstellung von Bekleidungsstücken oder Bandagen bekannt, bei dem eine Stofflage des Bekleidungsstücks bzw. der Bandage mit einem Verbund von Elastomer und Verstärkungsmaterial verbunden wird. Hierbei wird auf eine Elastomerschicht das Verstärkungsmaterial im flüssigen oder weichen, verformbaren Zustand aufgetragen, das nach dem Auftragen aushärtet. Die Elastomerschicht und das Verstärkungsmaterial werden über jeweils eine Düse aufgetragen, wobei die Düse für das Verstärkungsmaterial als ein 3D-Druckkopf ausgebildet ist.

Aus der US 2017/0151770 A1 ist es bekannt, mithilfe eines Druckkopfes schichtweise Elastomer auf eine Stofflage aufzubringen.

Aus der DE 10 2018 104 774 B3 ist eine auf der Haut tragbare Elektrode bekannt, die in ein Bekleidungsstück oder in eine Bandage integriert ist. Die Elektrode umfasst eine elektrisch leitende Hautkontaktschicht, die über einen elektrisch leitenden Klettverschluss mit einem Stromanschluss zu verbinden ist.

In der DE 10 2020 200 508 A1 wird ein Druckkopf für ein Drucksystem zur additiven Herstellung eines Faserverbundbauteils beschrieben. Der Druckkopf umfasst einen ersten Zufuhrabschnitt zum Zuführen von strangförmigem Thermoplastmaterial in den Druckkopf und einen zweiten Zufuhrabschnitt zum Zuführen von strangförmigem Fasermaterial oder Leitermaterial als Kernmaterial in den Druckkopf. Desweiteren sind eine Heizeinrichtung zum Plastifizieren des durch den ersten Zufuhrabschnitt zugeführten Thermoplastmaterials und eine Schneideinrichtung zum Durchtrennen des durch den zweiten Zufuhrabschnitt zugeführten Kernmaterials vorgesehen. Über eine Düse des Druckkopfes, die einen Extrusionskanal bildet, wird das Kernmaterial in das Thermoplastmaterial eingebettet.

Die US 2015/0 165 666 A1 offenbart eine Vorrichtung mit einer Düse zum Koextrudieren eines Polymerfadens und eines Faserfadens.

Die EP 3 130 444 A1 beschreibt einen 3D-Drucker, bei dem ein Verstärkungsfilament mit einem Kern und einem den Kern umgebenden Matrixmaterial in eine Extrusionsdüse eingeführt wird.

Aus der EP 0 187 270 A1 ist ein Verfahren zum Fixieren eines elastischen Bandes oder einer elastischen Folie auf flächigen Materialien bekannt. Durch Koextrusion oder sukzessive Extrusion wird ein Produkt hergestellt, das aus einer Hotmeltschicht und einer Schicht aus einem thermoplastischen Elastomermaterial besteht.

In der DE 197 44 527 A1 wird die Herstellung eines isolierten elektrischen Leiters im Extrusionsverfahren beschrieben. Die Extrusion erfolgt durch ineinandergesetzte, innere und äußere Düsen.

Die WO 2017/091154 A1 offenbart, einen elektrischen Leiter abschnittsweise mit einer Isolierung zu versehen, so dass der Leiter sich aus isolierten und aus nicht-isolierten Abschnitten zusammensetzt. Dieses Dokument offenbart auch ein Verfahren mit den Merkmalen des Oberbegriffs des Anspruchs 1.

Die US 2019/0009472 A1 offenbart eine Düse zum Auftragen eines Leiters 1502 und von Beschichtungsmaterial, das nach dem Beenden des Auftragungsvorgangs den Leiter einhüllt. Bei dem Beschichtungsmaterial handelt es sich um ein isolierendes Kunststoffmaterial.

Der Erfindung liegt die Aufgabe zugrunde, auf einfache und effiziente Weise Elastomer und ein in das Elastomer eingebettetes Kabel auf eine Stofflage eines Bekleidungsstücks oder einer Bandage aufzubringen.

Diese Aufgabe wird erfindungsgemäß mit den Merkmalen des unabhängigen Anspruchs 1 gelöst. Die abhängigen Ansprüche geben zweckmäßige Weiterbildungen an.

Mithilfe des erfindungsgemäßen Verfahrens kann in einem einzigen Verfahrensschritt Elastomer und ein Kabel auf eine Stofflage eines Bekleidungsstücks oder einer Bandage in der Weise aufgebracht werden, dass das Kabel teilweise oder vollständig von dem Elastomer umhüllt ist. Das Verfahren wird mithilfe einer Düse durchgeführt, die sowohl für das Auftragen des Elastomers als auch für das Auftragen des Kabels auf die Stofflage ausgebildet ist. Hierbei wird das Elastomer im verformbaren Zustand über die Düse aufgetragen und gleichzeitig auch das Kabel über diese Düse auf die Stofflage aufgetragen. Es genügt somit lediglich eine einzige Düse für den Auftrag sowohl von Elastomer als auch des Kabels. Das Kabel wird in eine Elastomerbahn auf der Stofflage gelegt, die von dem aus der Düse austretenden Elastomer erzeugt wird. Der Ausstoß von Elastomer und Kabel aus der gemeinsamen Düse erfolgt zeitgleich.

Nach Abschluss des Verfahrensschrittes ist es grundsätzlich nicht erforderlich, eine weitere Behandlung oder einen weiteren Verfahrensschritt durchzuführen. Das Kabel ist von dem Elastomer zumindest teilweise umhüllt, wodurch zum einen eine sichere und feste Verbindung zwischen dem Kabel und der Stofflage erreicht wird und zum andern das Kabel vor äußeren Einflüssen, beispielsweise Feuchtigkeit geschützt ist. Die Verbindung zwischen dem Elastomer und der Stofflage erfolgt dadurch, dass das Elastomer im verformbaren bzw. weichen, fließfähigen Zustand auf die Stofflage aufgetragen wird, so dass das Elastomer in die Stofflage eindringen kann. Nach dem Aushärten ist das Elastomer fest mit der Stofflage verbunden.

Bei dem Bekleidungsstück, das als Bestandteil die Stofflage aufweist, handelt es sich beispielsweise um ein Unterbekleidungsstück oder um ein Oberbekleidungsstück wie beispielsweise ein Hemd, ein T-Shirt, eine Hose wie zum Beispiel eine Sporthose, einen Büstenhalter oder dergleichen. Des Weiteren kommt auch ein Gürtel, ein Brustgurt oder eine Manschette in Betracht, die zum Beispiel über das Handgelenk oder das Fußgelenk gestülpt wird; auch derartige Gegenstände zählen im Sinne der Erfindung als Bekleidungsstück. Die Stofflage kann auch Bestandteil einer Bandage sein, insbesondere einer Gelenkbandage wie beispielsweise einer Kniebandage oder einer Ellbogenbandage.

Das Kabel ist vorteilhafterweise elektrisch leitfähig ausgebildet und dementsprechend in der Lage, elektrische Signale zu übertragen. Dies ermöglicht es, beispielsweise Elektroden oder Sensoren in das Bekleidungsstück bzw. die Bandage zu integrieren, wobei elektrische Signale von dem Sensor bzw. der Elektrode über das Kabel, welches an den Sensor bzw. die Elektrode angeschlossen ist, zu einer Auswerteeinheit übertragen werden können, in der die elektrischen Signale einer Auswertung unterzogen werden. Mithilfe des Sensors bzw. der Elektrode werden beispielsweise Vitalparameter des Körpers anhand von Spannungsänderungen der Haut oder einer Änderung des elektrischen Hautwiderstandes ermittelt. Umgekehrt ist es auch möglich, die Elektrode bzw. den Sensor als Signalgeber einzusetzen, über den elektrische Impulse, die in einem Impulsgeber erzeugt werden, auf die Haut übertragen werden, beispielsweise zur Muskelstimulation.

Gemäß einer weiteren vorteilhaften Ausführung treten das Elastomer und das Kabel über die gleiche Düsenöffnung aus der Düse aus. Es genügt somit grundsätzlich, dass die Düse nur eine Austrittsöffnung aufweist, über die sowohl das Elastomer als auch das Kabel austreten. Diese Ausführung hat den Vorteil, dass das Kabel bereits mit dem Austreten aus der Düsenöffnung zumindest teilweise von dem Elastomer umhüllt ist und bei einer Relativbewegung zwischen Düse und Stofflage eine Elastomerbahn mit integriertem Kabel erzeugt wird.

Alternativ ist es auch möglich, eine gemeinsame Düse für Kabel und Elastomer vorzusehen, die jedoch zwei Austrittsöffnungen besitzt, von denen eine Austrittsöffnung dem Kabel und die zweite Austrittsöffnung dem Elastomer zugeordnet sind. In diesem Fall treten das Kabel und das Elastomer über unterschiedliche Düsenöffnungen aus, die jedoch in der Weise angeordnet sein können, dass das Kabel in gewünschter Weise in das Elastomer auf der Stofflage eingebettet wird. Vorteilhafterweise ist die Austrittsöffnung für das Elastomer in Verfahrrichtung der Düse der Austrittsöffnung für das Kabel vorgelagert, so dass in Verfahrrichtung zuerst das Elastomer und darauffolgend das Kabel ausgebracht werden.

Mithilfe des Verfahrens kann gleichzeitig Elastomer und das Kabel aus der Düse austreten. Dieser Vorgang wird phasenweise durchgeführt. Das Kabel wird in einer weiteren Phase ohne Elastomer über die Düse auf die Stofflage aufgetragen. Dementsprechend wird das Ausbringen von Elastomer gestoppt, indem beispielsweise die Elastomerzufuhr zu der Düse unterbrochen wird. Das alleinige Ausbringen des Kabels aus der Düse hat den Vorteil, dass ein Kabelabschnitt frei von Elastomer auf der Stofflage aufliegt oder sich mit geringem Abstand zu der Stofflage befindet, so dass dieser Abschnitt an eine elektrische Baueinheit angeschlossen werden kann, insbesondere dem Sensor bzw. der Elektrode oder einer Auswerteeinheit. Es ist insbesondere möglich, zwei gegenüberliegende Enden des Kabels frei von Elastomer zu belassen, so dass ein Ende mit dem Sensor bzw. der Elektrode und das gegenüberliegende Ende mit der Auswerteeinheit verbunden werden können. Indem die Phase, in der nur das Kabel, jedoch kein Elastomer über die Düse aufgebracht wird, am Beginn oder am Ende eines Arbeitsschrittes liegt, genügt es, nur einen einzigen Arbeitsschritt mit einer Relativbewegung zwischen Kabel und Stofflage vorzusehen, um das Kabel phasenweise mit Elastomer auszubringen, so dass das Kabel vom Elastomer umhüllt ist, und phasenweise nur das Kabel, jedoch kein Elastomer auszubringen, so dass ein oder mehrere Abschnitte des Kabels frei von Elastomer liegen.

Dementsprechend weist das erfindungsgemäße Verfahren, bei dem Elastomer und ein in das Elastomer eingebettetes Kabel auf eine Stofflage eines Bekleidungsstücks oder einer Bandage aufgebracht wird, die folgenden Verfahrensschritte auf:
- das Elastomer wird mithilfe einer Düse im verformbaren Zustand auf die Stofflage aufgetragen,
- das Kabel wird über die gleiche Düse phasenweise gleichzeitig mit dem Elastomer in der Weise auf die Stofflage aufgetragen, dass das Elastomer das Kabel zumindest teilweise umhüllt,
- in einer anderen Phase wird nur das Kabel, jedoch kein Elastomer über die Düse auf die Stofflage aufgetragen.

Somit bezieht sich die Erfindung auf ein Verfahren zum Aufbringen von Elastomer und einem in das Elastomer eingebetteten Kabel auf eine Stofflage eines Bekleidungsstücks oder einer Bandage, wobei das Elastomer mithilfe einer Düse im verformbaren Zustand auf die Stofflage aufgetragen wird und das Kabel über die gleiche Düse phasenweise gleichzeitig mit dem Elastomer in der Weise auf die Stofflage aufgetragen wird, dass das Elastomer das Kabel zumindest teilweise umhüllt. In einer anderen, weiteren Phase wird nur das Kabel, jedoch kein Elastomer über die Düse auf die Stofflage aufgetragen. Ein aus der Düse bereits ausgetretenes Kabelende wird relativ zur Stofflage fixiert. Die Fixierung kann entweder unmittelbar auf der Stofflage erfolgen oder an einem ortsfesten Gegenstand, beispielsweise einem Tisch, auf dem die Stofflage aufliegt. Die Fixierung des aus der Düse ausgetretenen Kabelendes erfolgt vor dem Arbeitsschritt mit zumindest phasenweise gleichzeitigem Auftragen von Elastomer und Kabel. Aufgrund der Fixierung kann bei einer Relativbewegung zwischen Düse und Stofflage das Kabel aus der Düsenöffnung in der Düse herausgezogen werden, sobald beispielsweise die Düse sich über die Stofflage bewegt. Diese Vorgehensweise hat den Vorteil, dass auf eine Antriebseinrichtung zum Antreiben des Kabels verzichtet werden kann.

Zusätzlich ist es auch möglich, dass das Kabel mithilfe einer Antriebseinrichtung mit einer Geschwindigkeit angetrieben wird, die der Relativgeschwindigkeit zwischen der Düse und der Stofflage entspricht. Die Antriebseinrichtung kann entweder der Düse vorgelagert sein, bevor das Kabel mit Antrieb in die Düse hineingeführt wird, oder der Düse nachgeordnet sein, so dass das Kabel mithilfe der Antriebseinrichtung aus der Düse herausgezogen wird. Die Antriebseinrichtung hat den Vorteil, dass, in einer nicht beanspruchten Ausführungsform, das aus der Düse bereits ausgetretene Kabelende nicht fixiert werden muss.

Gemäß noch einer weiteren vorteilhaften Ausführung ist das verwendete Kabel in Kabellängsrichtung elastisch ausgebildet und weist eine verhältnismäßig hohe Elastizität auf, insbesondere in der Weise, dass das Kabel auf das mindestens 1.6fache elastisch dehnbar ist. Erst bei Dehnungen, die größer sind als das 1.6fache, wird der linear-elastische Bereich des Kabels verlassen. Bis zu dieser Dehnung jedoch ist ein linearelastisches Verhalten des Kabels bei einer Dehnung in Kabellängsrichtung gewährleistet. Die Elastizität des Kabels erlaubt es, dass Stoffdehnungen auch von dem Kabel mit ausgeführt werden können, wodurch das Traggefühl des Bekleidungsstücks oder der Bandage, das bzw. die mit dem Kabel versehen ist, nicht beeinträchtigt wird. Stoffdehnungen können auch von dem Elastomer mit ausgeführt werden, mit dem das Kabel zumindest teilweise umhüllt ist und das das Kabel an der Stofflage hält.

Die Umhüllung des Kabels durch das Elastomer erfolgt vorzugsweise derart, dass das Kabel auf der der Stofflage abgewandten Seite vollständig von Elastomer bedeckt ist. Das Kabel kann gegebenenfalls unmittelbar auf der Stofflage aufliegen. Alternativ ist es auch möglich, dass das Kabel ringsum von Elastomer umhüllt ist, also auch auf der der Stofflage zugewandten Seite.

Gemäß einer weiteren vorteilhaften Ausführung wird ein elastisches Kabel verwendet, das aus einem Gummikern und einem umhüllenden Metallnetz besteht. Der Gummikern ist Träger des umhüllenden Metallnetzes und erlaubt eine elastische Dehnung in Längsrichtung. Das Metallnetz, welches den Gummikern umhüllt, ist elektrisch leitfähig und ermöglicht eine elektrische Signalübertragung, wobei zugleich die Stabilität des Kabels durch das Metallnetz verbessert ist. Bei einer Dehnung in Kabellängsrichtung wird auch das Metallnetz gedehnt. Sobald sich der Gummikern wieder elastische zusammenzieht, wird auch das Metallnetz wieder zusammengezogen.

Als Elastomer wird beispielsweise ein thermoplastisches Elastomer verwendet, das bei Erwärmung weich oder flüssig wird und mit dem Abkühlen aushärtet. Im erwärmten Zustand kann das Elastomer über die Düse aufgetragen werden, woraufhin das Elastomer aushärtet und hierbei die feste Verbindung mit der Stofflage eingeht und außerdem das Kabel schützt und an der Stofflage sichert. Im ausgehärteten Zustand besitzt das Elastomer ein elastisches Verhalten. Als Elastomer kann zum Beispiel ein Silikon verwendet werden.

Optional wird auf das Elastomer nach dem Auftragen auf die Stofflage und im noch nicht ausgehärteten Zustand ein Beflockungsmaterial aufgebracht, wodurch das Tragegefühl verbessert werden kann. Das Beflockungsmaterial ist in der Lage, Feuchtigkeit aufzunehmen.

Es wird auch ein Bekleidungsstück oder eine Bandage mit einer Stofflage beschrieben, die Träger eines Kabels ist, welches mithilfe von Elastomer mit der Stofflage verbunden ist. Das Kabel ist in der vorbeschriebenen Weise ausgebildet, vorzugsweise elektrisch leitfähig und in Kabellängsrichtung elastisch. Das Bekleidungsstück bzw. die Bandage wird vorteilhafterweise nach dem vorbeschriebenen Verfahren hergestellt.

Mindestens ein Kabelende, vorzugsweise beide Kabelenden liegen frei von Elastomer, wobei der zwischen den Kabelenden liegende Kabelabschnitt mit Elastomer bedeckt ist. In dieser Ausführung ist das Kabel fest mit der Stofflage verbunden, zugleich kann zumindest ein Kabelende, vorzugsweise beide Kabelenden, beispielsweise mit einem Sensor oder einer Elektrode und einer Auswerteeinheit oder einer Signalerzeugungseinheit verbunden werden. Bei dem Bekleidungsstück handelt es sich vorteilhafterweise um ein vorbeschriebenes Bekleidungsstück, gleiches gilt für die Bandage.

Das Elastomer und das Kabel können sowohl auf der dem Körper zugewandten Innenseite des Bekleidungsstücks als auch auf der dem Körper abgewandten Außenseite des Bekleidungsstücks angeordnet sein.

Eine Düse zum gemeinsamen Auftragen von Elastomer und Kabel auf eine Gewebelage wird ebenfalls beschrieben. Das Bekleidungsstück bzw. die Bandage wird nach dem vorbeschriebenen Verfahren unter Verwendung der Düse hergestellt. Die Düse weist einen Düsenkörper auf, in den zwei Zufuhröffnungen eingebracht sind, von denen eine erste Zufuhröffnung für die Zufuhr von Elastomer und die zweite Zufuhröffnung für die Zufuhr des Kabels dienen. Gegebenenfalls können das Elastomer und das Kabel über eine gemeinsame Zufuhröffnung zugeführt werden.

Das Elastomer und das Kabel können über die gemeinsame Düse gleichzeitig oder zeitlich versetzt auf die Stofflage aufgetragen werden.

Der Düsenkörper weist einen Düsenkörperinnenraum auf, in den die erste und die zweite Zufuhröffnung münden. Außerdem weist die Düse eine gemeinsame Austrittsöffnung sowohl für das Elastomer als auch für das Kabel auf. Möglich ist auch eine Ausführung mit jeweils einer Austrittsöffnung für Elastomer und das Kabel. Innerhalb des Düsenkörperinnenraums gelangt das Kabel in Kontakt mit dem Elastomer und wird von dem Elastomer benetzt. Wenn das unter Druck stehende Elastomer über die Austrittsöffnung aus der Düse austritt, wird durch die Haftung des Kabels an das Elastomer auch das Kabel mit einer Kraft aus der Austrittsöffnung der Düse hinausbewegt. Diese Kraft kann ausreichend sein, um einen kontinuierlichen Austritt des Kabels aus der Düse zu bewerkstelligen.

Es kann sich an oder in der Düse eine Antriebseinrichtung für das Kabel finden, welche das Kabel antreibt, wobei je nach Positionierung der Antriebseinrichtung entweder das Kabel in die Düse hineinbefördert oder aus der Düse herausgezogen wird.

Es kann in den Düsenkörper der Düse ein röhrenförmiges Kabelführungsteil integriert sein, durch das das Kabel geführt ist. Das Kabelführungsteil ist durch den Düsenkörperinnenraum geführt und stellt sicher, dass der Kontakt zwischen dem Elastomer und dem Kabel erst nach dem Austritt aus der Düse erfolgt. Dies hat den Vorteil, dass das Kabel innerhalb der Düse nicht am Elastomer haftet und das Ausbringen des Kabels aus der Düse ungehindert vom Elastomer und mit geringen Vorschubkräften erfolgt. Die Austrittsöffnung des Kabelführungsteils liegt zweckmäßigerweise in oder benachbart zu der Austrittsöffnung der Düse.

Das Ende des röhrenförmigen Kabelführungsteils kann in die Austrittsöffnung hineinragen. Das Kabelführungsteil ist vorteilhafterweise einteilig mit dem Düsenkörper ausgebildet.

Weitere Vorteile und zweckmäßige Ausführungen sind den weiteren Ansprüchen, der Figurenbeschreibung und den Zeichnungen zu entnehmen. Es zeigen:
- Fig. 1: einen Schnitt durch eine Düse zum gemeinsamen Auftrag von Elastomer und einem Kabel auf eine Stofflage,
- Fig. 2: die Düse gemäß Fig. 1, jedoch mit einer zusätzlichen Antriebseinrichtung zum Antreiben des Kabels durch die Düse,
- Fig. 3: in vergrößerter Darstellung die Stofflage mit Elastomer und Kabel,
- Fig. 4: einen Schnitt durch ein elektrisch leitfähiges Kabel,
- Fig. 5: einen Schnitt durch eine Düse zum gemeinsamen Auftrag von Elastomer und einem Kabel in einer weiteren Ausführung.

In den Figuren sind gleiche Bauteile mit gleichen Bezugszeichen versehen.

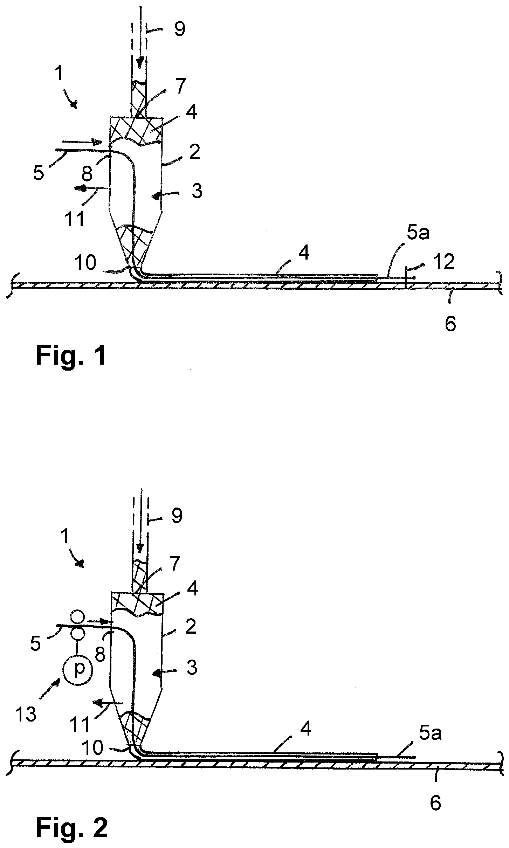

In Fig. 1 ist eine Düse 1 dargestellt, die einen Düsenkörper 2 aufweist, wobei über den Düsenkörperinnenraum 3 im Düsenkörper 2 ein darin befindliches Elastomer 4 und ein Kabel 5 auf eine Stofflage 6 aufgetragen werden können. Die Stofflage 6 liegt vorzugsweise horizontal auf einem Arbeitstisch auf. Die Düse 1 wird mithilfe einer geeigneten Düsenvorrichtung über die Oberseite der Stofflage 6 hinwegbewegt, und zugleich werden das Elastomer 4 und das Kabel 5 aus der Düse 1 ausgebracht und auf die Oberseite der Stofflage 6 aufgetragen.

Das Kabel 5 ist vorzugsweise ein elektrisch leitfähiges Kabel, das zur elektrischen Signalübertragung eingesetzt wird. Das Kabel 5 ist mithilfe des Elastomers 4 fest mit der Stofflage 6 verbunden. Das Kabel 5 wird insbesondere von dem Elastomer 4 umhüllt und ist hierdurch vor äußeren Einflüssen wie beispielsweise Feuchtigkeit geschützt. Das Kabel 5 ist vorteilhafterweise längselastisch ausgebildet und besitzt insbesondere eine Elastizität, gemäß der das Kabel 5 auf das mindestens 1.6fache linear-elastisch dehnbar ist. Mithilfe des Kabels 5 können beispielsweise elektrische Signale zwischen einer Elektrode oder einem Sensor, die bzw. der in die Stofflage 6 integriert ist, und einer Auswerteeinheit oder einer Signalerzeugungseinheit übertragen werden. Über den Sensor bzw. die Elektrode können zum Beispiel Vitalparameter einer Person aufgenommen werden, welche ein Bekleidungsstück und eine Bandage mit der gezeigten Stofflage 6 trägt. Es ist auch möglich, über das Kabel 5 Stromimpulse auf eine Elektrode in der Stofflage 6 zu übertragen, über die einzelne Muskelpartien gezielt stimuliert werden.

Bei dem Bekleidungsstück, in das die Stofflage 6 integriert wird, handelt es sich beispielsweise um ein Sportbekleidungsstück wie ein T-Shirt oder eine Sporthose. Im Fall einer Bandage, in die die Stofflage 6 integriert wird, handelt es sich insbesondere um eine manschettenförmige Gelenkbandage, zum Beispiel eine Kniebandage oder eine Ellbogenbandage.

An dem Düsenkörper 2 befindet sich eine erste, obenliegende Zufuhröffnung 7 für die Zufuhr des Elastomers 4 über eine Zufuhrleitung 9. In die Seitenwand des Düsenkörpers 2 ist eine zweite Zufuhröffnung 8 eingebracht, über die das Kabel 5 in den Düsenkörperinnenraum 3 eingeleitet wird. Der Düsenkörper 2 verjüngt sich nach unten hin, wobei an der untenliegenden Spitze des Düsenkörpers 2 eine gemeinsame Austrittsöffnung 10 für den Austritt von Elastomer 4 und Kabel 5 angeordnet ist.

Über die Zufuhrleitung 9 wird unter Druck stehendes, fließfähiges Elastomer 4 in den Düsenkörperinnenraum 3 eingeleitet, in den auch über die zweite Zufuhröffnung 8 im Seitenbereich des Düsenkörpers 2 das Kabel 5 eingeführt wird. Im Düsenkörperinnenraum 3 haftet sich das Elastomer 4 an das Kabel 5 an, woraufhin Elastomer 4 und das Kabel 5 gemeinsam über die untenliegende Austrittsöffnung 10 austreten und auf die Oberseite der Stofflage 6 aufgebracht werden. Die Zufuhrrichtungen von Elastomer durch die Zufuhrleitung 9 und des Kabels 5 seitlich in den Düsenkörperinnenraum 3 hinein sind jeweils mit Pfeilen gekennzeichnet.

Die Düse 1 wird in der Weise über die Stofflage 6 geführt, dass die untenliegende Austrittsöffnung 10 einen kleinen Abstand zur Oberseite der Stofflage 6 aufweist. Die Düse 1 befindet sich vorteilhafterweise an einer Vorrichtung, mittels der die Düse 1 horizontal in Pfeilrichtung 11 über die feststehende Stofflage 6 bewegt wird. Beim Austritt aus der Austrittsöffnung 10 bildet sich eine Raupe auf der Oberseite der Stofflage 6, bestehend aus Elastomer 4 und Kabel 5, das vorzugweise vollständig von dem Elastomer 4 umhüllt ist. Das Elastomer 4 wird im fließfähigen Zustand aufgebracht und kann in die Poren der Stofflage 6 eindringen. Nach dem Aushärten ist eine feste Verbindung von Elastomer 4 einschließlich dem Kabel 5 mit der Stofflage 6 gegeben.

Das Kabelende 5a des Kabels 5 wird zu Beginn eines Arbeitsschrittes, nachdem es durch die Düse 2 durchgezogen wurde und über die Austrittsöffnung 10 ausgetreten ist, ohne Elastomer 4 an der Stofflage 6 oder einem Arbeitstisch, auf dem die Stofflage 6 aufliegt, fixiert. Hierzu ist ein Fixierelement 12 vorgesehen, beispielsweise eine Klammer oder dergleichen, das an dem Kabelende 5 angreift. Sobald die Düse 2 in Pfeilrichtung 11 über die Stofflage 6 bewegt wird, wird das Kabel 5 aufgrund seiner Befestigung über das Fixierelement 12 in den Düsenkörper 2 hineingezogen und über die Austrittsöffnung 10 herausgezogen.

Das von Elastomer 4 freie Kabelende 5a eignet sich zum Anschluss beispielsweise an einen Sensor, an eine Elektrode oder auch eine Auswerteeinheit oder Signalerzeugungseinheit.

In entsprechender Weise kann ein gegenüberliegendes, zweites Kabelende auch frei von Elastomer gehalten werden. Die Elastomerzufuhr wird hierfür kurz unterbrochen.

Das Elastomer 4 wird insbesondere im erwärmten, fließfähigen Zustand und unter Druck über die Zufuhrleitung 9 in den Düsenkörperinnenraum 3 und weiter über die Austrittsöffnungen 10 auf die Stofflage 6 aufgebracht. Mit dem Abkühlen härtet das Elastomer 4 aus, wobei auch im ausgehärteten Zustand ein elastisches Verhalten des Elastomers gegeben ist. Auch das Kabel 5 ist elastisch ausgebildet, so dass sich die Stofflage auch nach dem Auftragen von Elastomer 4 und Kabel 5 elastisch dehnen und wieder zusammenziehen kann.

In Fig. 2 ist eine Ausführungsvariante dargestellt, in der die Düse 1 grundsätzlich den gleichen Aufbau wie bei Fig. 1 aufweist, jedoch eine zusätzliche Antriebseinrichtung 13 zum Antreiben des Kabels 5 vorgesehen ist. Die Antriebseinrichtung 13 umfasst einen Elektromotor, eine angetriebene Rolle und eine passiv mitdrehende Rolle, wobei die angetriebene Rolle von dem Elektromotor angetrieben wird und das Kabel 5 zwischen den beiden Rollen hindurchgeführt ist. Die Antriebseinrichtung 13 ist im Düsenkörper 2 vorgelagert, so dass das Kabel 5 mit Antrieb in den Düsenkörperinnenraum 3 hineinbefördert wird. Grundsätzlich möglich ist auch eine Ausbildung am Austritt aus dem Düsenkörper 2, um das Kabel 5 mithilfe der Antriebseinrichtung aus dem Düsenkörperinnenraum 4 herauszuziehen. Die Antriebseinrichtung 13 kann an dem Düsenkörper 2 oder einer den Düsenkörper 2 haltenden Vorrichtung befestigt werden.

Die Geschwindigkeit, mit der das Kabel 5 von der Antriebseinrichtung 13 angetrieben wird, entspricht insbesondere der Relativgeschwindigkeit zwischen Düse 1 und Stofflage 6. Dies stellt einen kontinuierlichen, gleichmäßigen Austritt von Kabel 5 und Elastomer 4 über die Austrittsöffnung 10 im unteren Bereich der Düse 1 sicher. Das Kabel 5 kann hierbei ohne Dehnungen oder Stauchungen auf die Stofflage 6 aufgetragen werden. Des Weiteren ist es vorteilhaft, dass auf eine Fixierung des Kabels 5 an der Stofflage 6 oder einer die Stofflage 6 tragenden Vorrichtung verzichtet werden kann.

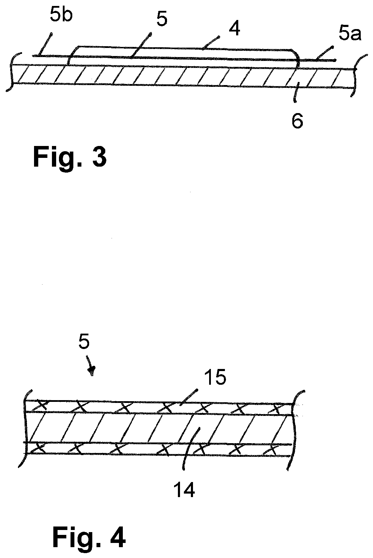

In Fig. 3 ist die Stofflage 6 mit aufgebrachtem, raupenförmigem Elastomer und darin aufgenommenem Kabel 5 in vergrößerter Darstellung gezeigt. Zu beiden Seiten des Elastomers 4 ragt das Kabel mit Kabelenden 5a, 5b heraus, die somit blank und frei von Elastomer liegen und zum Anschluss an einen Sensor, einer Elektrode oder einer Auswerteeinheit oder Signalerzeugungseinheit genutzt werden können.

Fig. 4 zeigt einen Längsschnitt durch das Kabel 5, das aus einem Gummikern oder einer Gummiseele 14 und einem umhüllenden Metallnetz 15 besteht. Das Metallnetz 15 ist als ein Drahtgeflecht ausgebildet, das elektrisch leitend ist und elektrische Signale übertragen kann. Der innenliegende Gummikern 14 kann stark gedehnt werden und zieht sich nach linear-elastischer Dehnung wieder in den Ausgangszustand zurück. Das Metallnetz 15 in Form des Drahtgeflechts kann ebenfalls entsprechende Dehnungen ausüben und wird entweder aufgrund eines eigenen elastischen Verhaltens oder über den Gummikern 14 nach Wegfall äußerer Kräfte wieder in den zurückgezogenen Ausgangszustand gebracht.

Die linear-elastische Dehnung des Kabels 5 beträgt mindestens 60 % und somit das 1.6fache gegenüber dem ungedehnten Ausgangszustand.

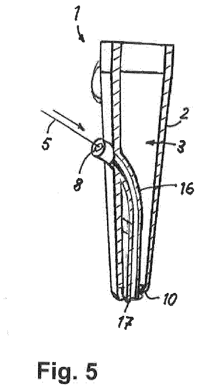

In Fig. 5 ist eine Düse 1 in einer Ausführungsvariante dargestellt, bei der in den Düsenkörper 2 der Düse 1 ein röhrenförmiges Kabelführungsteil 16 integriert ist, durch das das Kabel 5 geführt ist. Das Kabelführungsteil ist durch den Düsenkörperinnenraum 3 geführt. Die Zufuhröffnung 8, über die das Kabel 5 in das Kabelführungsteil 16 hineingeführt wird, befindet sich an einem vorderen Ende des Kabelführungsteil 16 im Seitenbereich des Düsenkörpers 2. Das untere Ende des gekrümmt ausgeführten Kabelführungsteils 16 ragt in die Austrittsöffnung 10 der Düse 1 hinein, wobei die Austrittsöffnung 17 des Kabelführungsteils 16 in der Austrittsöffnung 10 der Düse 1 liegt. Das Elastomer kann über den ringförmigen Bereich zwischen der Außenwand des Kabelführungsteils 16 und der Innenwand der Austrittsöffnung 10 austreten.

Das Kabelführungsteil 16 ist einteilig mit dem Düsenkörper 2 ausgebildet.

## Patentansprüche

1. Verfahren, bei dem Elastomer (4) und ein in das Elastomer (4) eingebettetes Kabel (5) auf eine Stofflage (6) eines Bekleidungsstücks oder einer Bandage aufgebracht werden, **gekennzeichnet durch** die folgenden Verfahrensschritten:
- das Elastomer (4) wird mithilfe einer Düse (1) im verformbaren Zustand auf die Stofflage (6) aufgetragen,
- das Kabel (5) wird über die gleiche Düse (1) phasenweise gleichzeitig mit dem Elastomer (4) in der Weise auf die Stofflage (6) aufgetragen, dass das Elastomer (4) das Kabel (5) zumindest teilweise umhüllt,
- in einer anderen Phase wird nur das Kabel (5), jedoch kein Elastomer (4) über die Düse (1) auf die Stofflage (6) aufgetragen,
wobei ein aus der Düse (1) bereits ausgetretenes Kabelende (5a, 5b) relativ zur Stofflage (6) fixiert wird,
wobei die Fixierung des aus der Düse (1) ausgetretenen Kabelendes (5a, 5b) vor dem Arbeitsschritt mit zumindest phasenweise gleichzeitigem Auftragen von Elastomer (4) und Kabel (5) erfolgt.

2. Verfahren nach Anspruch 1,
**dadurch gekennzeichnet,**
**dass** das Elastomer (4) und das Kabel (5) über die gleiche Düsenöffnung aus der Düse (1) austreten.

3. Verfahren nach Anspruch 1 oder 2,
**dadurch gekennzeichnet,**
**dass** die andere Phase, in der nur das Kabel (5), jedoch kein Elastomer (4) über die Düse (1) auf die Stofflage (6) aufgebracht wird, am Beginn oder am Ende eines Arbeitsschrittes liegt, in welchem das Kabel (5) aus der Düse (1) austritt.

4. Verfahren nach einem der Ansprüche 1 bis 5,
**dadurch gekennzeichnet,**
**dass** das Kabel (5) mithilfe einer Antriebseinrichtung (13) mit einer Geschwindigkeit angetrieben wird, die der Relativgeschwindigkeit zwischen der Düse (1) und Stofflage (6) entspricht.

5. Verfahren nach einem der Ansprüche 1 bis 4,
**dadurch gekennzeichnet,**
**dass** das Kabel (5) elastisch ausgebildet ist und eine Elastizität aufweist, gemäß der das Kabel (5) auf das mindestens 1.6fache elastisch dehnbar ist.

6. Verfahren nach einem der Ansprüche 1 bis 5,
**dadurch gekennzeichnet,**
**dass** ein elastisches Kabel (5) verwendet wird, das aus einem Gummikern (14) und einem umhüllenden Metallnetz (15) besteht.

7. Verfahren nach einem der Ansprüche 1 bis 6,
**dadurch gekennzeichnet,**
**dass** das Kabel (5) elektrisch leitfähig ausgebildet ist.

## Claims

1. Method wherein elastomer (4) with a cable (5) embedded in the elastomer (4) is applied onto a fabric layer (6) of a garment or a bandage **characterized by** the following method steps:
- the elastomer is applied to the fabric layer (6) in a deformable state via a nozzle (1),
- the cable (5) is applied to the fabric layer (6) via the same nozzle (1) in phases at the same time together with the elastomer (4) in such a way that the elastomer (4) surrounds the cable (5) at least partially,
- in another phase only the cable (5) but no elastomer (4) is applied via the nozzle (1) to the fabric layer (6),
wherein an end (5a, 5b) of the cable (5) that has already exited the nozzle (1) is fixed relative to the fabric layer (6),
and the fixing of the end (5a, 5b) of the cable (5) which has exited the nozzle (1) occurs before the method step in which the elastomer (4) and the cable (5) are applied at least in phases at the same time.

2. Method according to claim 1, **characterized in that** the elastomer (4) and the cable (5) exit the nozzle (1) via the same nozzle opening.

3. Method according to claim 1 or 2, **characterized in that** the other phase, in which only the cable (5) but no elastomer (4) is applied via the nozzle (1) to the fabric layer (6) is at the beginning or at the end of a method step in which the cable (5) exits the nozzle (1).

4. Method according to any of claims 1 to 3, **characterized in that** the cable (5) is moved by a drive arrangement (13) at a speed which corresponds to the relative speed between the nozzle (1) and the fabric layer (6).

5. Method according to any of claims 1 to 4, **characterized in that** the cable (5) is elastic with an elasticity with which the cable (5) is expandable by a value of at least 1.6 times.

6. Method according to claim 5, **characterized in that** the elastic cable (5) used consists of a rubber core (14) and a surrounding metal net (15).

7. Method according to any of claims 1 to 6, wherein the cable (5) is electrically conductive.

## Revendications

1. Procédé dans lequel un élastomère (4) et un câble (5) noyé dans l'élastomère (4) sont appliqués sur une couche de tissu (6) d'un vêtement ou d'un bandage, **caractérisé par** les étapes de procédé suivantes:
- l'élastomère (4) est appliqué sur la couche de matériau (6) dans un état déformable à l'aide d'une buse (1),
- le câble (5) est appliqué sur la couche de matériau (6) par phases simultanément avec l'élastomère (4) via la même buse (1) de telle sorte que l'élastomère (4) enveloppe au moins partiellement le câble (5),
- dans une autre phase, seul le câble (5), mais pas d'élastomère (4), est appliqué sur la couche de matériau (6) via la buse (1),
dans lequel une extrémité de câble (5a, 5b) déjà sortie de la buse (1) est fixée par rapport à la couche de matériau (6), dans lequel l'extrémité du câble (5a, 5b) sortant de la buse (1) est fixée avant l'étape de travail avec application simultanée d'élastomère (4) et de câble (5) au moins par phases.

2. Procédé selon la revendication 1,
**Caractérisé en ce que**
l'élastomère (4) et le câble (5) sortent de la buse (1) par la même ouverture de buse.

3. Procédé selon la revendication 1 ou 2,
**Caractérisé en ce que**
l'autre phase, dans laquelle seul le câble (5), mais aucun élastomère (4), est appliqué sur la couche de matériau (6) via la buse (1), se situe au début ou à la fin d'une étape de travail dans lequel le câble (5) sort de la buse (1).

4. Procédé selon l'une des revendications 1 à 3,
**caractérisé en ce que**
le câble (5) est entraîné au moyen d'un dispositif d'entraînement (13) à une vitesse qui correspond à la vitesse relative entre la buse (1) et la couche de matériau (6).

5. Procédé selon l'une des revendications 1 à 4,
**caractérisé en ce que**
le câble (5) est conçu de manière élastique et possède une élasticité selon laquelle le câble (5) peut être étiré élastiquement jusqu'à au moins 1,6 fois.

6. Procédé selon l'une des revendications 1 à 5,
**caractérisé en ce que**
on utilise un câble élastique (5) constitué d'une âme en caoutchouc (14) et d'un filet métallique enveloppant (15).

7. Procédé selon l'une des revendications 1 à 6,
**caractérisé en ce que**
le câble (5) est électriquement conducteur.
